# EUROPEAN PATENT APPLICATION

(11) **EP 4 806 554 A1**
(43) Date of publication of application: **16.09.2026**
(21) Application number: 25185355.2
(22) Date of filing: 25.06.2025
(51) Int. Cl.: B01L 3/00

(54) **CLOSURE SYSTEM FOR PROCESSING BIOLOGICAL SAMPLES**

(30) Priority: 11.03.2025 PT 2025012105
(71) Applicant: Sevlaires Plásticos, Lda., 2430-076 Marinha Grande (PT)
(72) Inventor: FORTUNATO GOMES, José, MARINHA GRANDE (PT)
(74) Representative: Pereira da Cruz, Joao

(57) **Abstract**

The present invention relates to closure systems for the safe handling of biological samples, specifically for collecting, preserving and transporting procedures.

The object of this invention is a closure system for processing biological samples, comprising a first container and a second container configured for fluid storage; a closure assembly comprising a single-piece male cap element and a single-piece female cap element, said male cap element comprising one rupturable membrane; said female cap element comprising a perforation structure configured to rupture said membrane upon actuation; and a locking mechanism configured to prevent the fluid communication between said first and second containers upon rupture of said membrane.

The proposed invention aims to reduce the risk of leaks or accidental exposure to harmful vapors and to prevent fixative liquid back flow, while simultaneously ensuring the safety for operators handling hazardous fixatives of biological samples.

## Description

### FIELD OF THE INVENTION

The present invention relates to closure systems for the safe handling of biological samples, specifically for collecting, preserving and transporting procedures.

### PRIOR ART

Collecting and preserving biological samples in laboratory and medical environments often requires the use of chemical fixatives, such as formaldehyde, to maintain tissue integrity before analysis. However, formaldehyde is a hazardous substance, classified as a carcinogen and respiratory irritant, necessitating strict containment measures to protect operators from inhalation exposure, skin contact, and accidental spills.

To minimize the risk of operator exposure, several closed-circuit containment systems have been developed to prevent direct contact between users and fixative liquids. However, existing solutions still present significant safety concerns, particularly in terms of vapor containment, fluid leakage, and uncontrolled backflow.

Some existing systems utilize male and female closure elements that are rotated into alignment, where holes in both elements match to allow fluid transfer between containers. While these designs enable controlled fluid movement, they exhibit critical safety flaws, such as the increased risk of vapor escape, since the holes remain exposed during alignment, fixative fumes may leak, increasing the risk of inhalation exposure; and also increased potential for spillage due to misalignment, considering that if holes are not precisely aligned, the user may experience unexpected leaks, drips, or unintended exposure to the hazardous liquid.

Another type of existing systems uses a perforation mechanism, where a piercing structure in the female cap element ruptures a membrane in the male cap element upon threading engagement. This method ensures an initial sealed system before perforation but introduces new safety risks, such as the permanent fluid communication after perforation - once the membrane is ruptured, the fluid transfer channel remains permanently open, allowing potential backflow of hazardous fixative liquid into the first container (1).

The present invention aims to reduce the risk of leaks or accidental exposure and to prevent fixative liquid back flow, while ensuring the safety for operators handling formaldehyde and other hazardous fixatives of biological samples.

### SUMMARY OF THE INVENTION

The object of this invention is a closure system (100) for processing biological samples, comprising:
- a first container (1) and a second container (2) configured for fluid storage;
- a closure assembly comprising a single-piece male cap element (10) and a single-piece female cap element (20), said male cap element (10) comprising attaching means (11) for closing said first container (1), means for coupling (12) with the female cap element (20) and one rupturable membrane (13); said female cap element (20) comprising attaching means (21) for closing said second container (2), means for coupling (22) with the male cap element (10), a perforation structure (23) configured to rupture said membrane (13) upon actuation, and a plurality of holes (30) to allow fluid communication between said first (1) and second containers (2); and
- a locking mechanism configured to prevent the fluid communication between said first (1) and second containers (2) upon rupture of said membrane (13), wherein said male cap element (10) comprises a membrane support structure (14) and the female cap element (20) comprises a locking plate (24), being said locking plate (24) configured to lock onto said membrane support structure (14) upon actuation to seal said rupture membrane (13) and block the fluid communication between said first (1) and second containers (2).

In an advantageous aspect of the present invention, the membrane support structure (14) of the male cap element (10) is permanently connected to the membrane (13) and comprises a rupture zone configured to receive the perforation structure (23) upon the rupturing of the membrane (13). In addition, by supporting the membrane (13) around said rupture zone, the membrane support structure (14) ensures that the hole formed upon the rupturing of the membrane (13) is contained within the rupture zone of the membrane support structure (14), to prevent fluid leaking and to limit the channel for fluid communication between the first (1) and second containers (2), including backflow.

In another advantageous aspect of the present invention, the locking plate (24) is configured to seal the hole of the ruptured membrane (13) once the perforation structure (23) is fully inserted through the membrane (13) into the rupture zone of the membrane support structure (14), wherein the locking plate (24) contacts the membrane support structure (14) around the hole formed by the rupturing of the membrane (13), thus providing a tight seal to block the fluid communication between said first (1) and second containers (2). In the locked position, the hole of the ruptured membrane (13) is sealed by the fully inserted perforation structure (23) and by the locking plate (24), which is locked onto the membrane support structure (14). Preferably, the membrane support structure (14) comprises a circular ring.

In a preferred embodiment of the present invention, the male cap element (10) comprises a first tubular portion with an open top end and a closed bottom end sealed by the rupturable membrane (13), having said means for coupling (12) with the female cap element (20) located on an exterior surface of said first tubular portion; the female cap element (20) comprises a second tubular portion with an open top end and a base located at the bottom end, having said means for coupling (22) with the male cap element (10) located on an interior surface of said second tubular portion; and the base of the female cap element (20) comprises the perforation structure (23) and said plurality of holes (30) for fluid communication between the first (1) and second containers (2).

### DESCRIPTION OF THE FIGURES

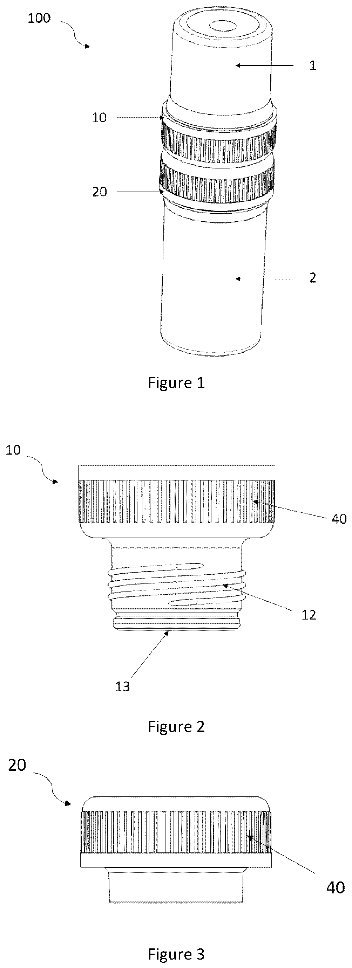

Figure 1 - perspective view of the closure system (100) for processing biological samples, comprising a first container (1) for storing fixative liquids and a second container (2) for receiving biological samples; a closure assembly comprising a single-piece male cap element (10) and a single-piece female cap element (20).
Figure 2 - side view of the male cap element (10), comprising means for coupling (12) with the female cap element (20), a rupturable membrane (13) and gripping means (40) comprising a plurality of grooves radially positioned along the external surface of the top portion of the open top end of the tubular portion of said male cap element (10).
Figure 3 - side view of the female cap element (20), comprising gripping means (40) comprising a plurality of grooves radially positioned along the external surface of the top portion of the open top end of the tubular portion of said female cap element (20).

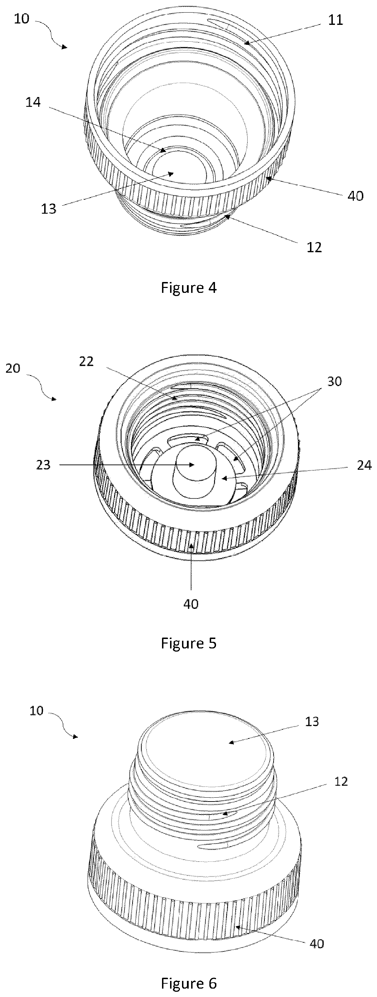

Figure 4 - perspective view of the male cap element (10), comprising means for coupling (12) with the female cap element (20), a rupturable membrane (13), a circular ring-like membrane support structure (14) and gripping means (40).
Figure 5 - perspective view of the female cap element (20), comprising means for coupling (22) with the male cap element (10), a central protruding pillar perforation structure (23), a locking plate (24) positioned at the bottom and around the central protruding pillar, a plurality of holes (30) radially positioned around the locking plate (24) and gripping means (40).
Figure 6 - another angle of a perspective view of the male cap element (10), comprising means for coupling (12) with the female cap element (20), a rupturable membrane (13) and gripping means (40).

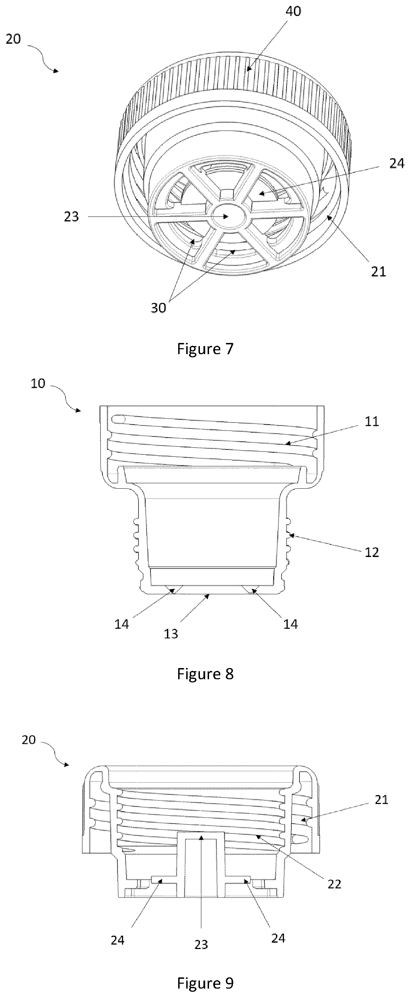

Figure 7 - another angle of a perspective view of the perspective view of the female cap element (20), comprising a central protruding pillar perforation structure (23), a locking plate (24) positioned at the bottom and around the central protruding pillar, a plurality of holes (30) radially positioned around and below the locking plate (24), and gripping means (40).
Figure 8 - vertical cross-section of the male cap element (10).
Figure 9 - vertical cross-section of the female cap element (20).

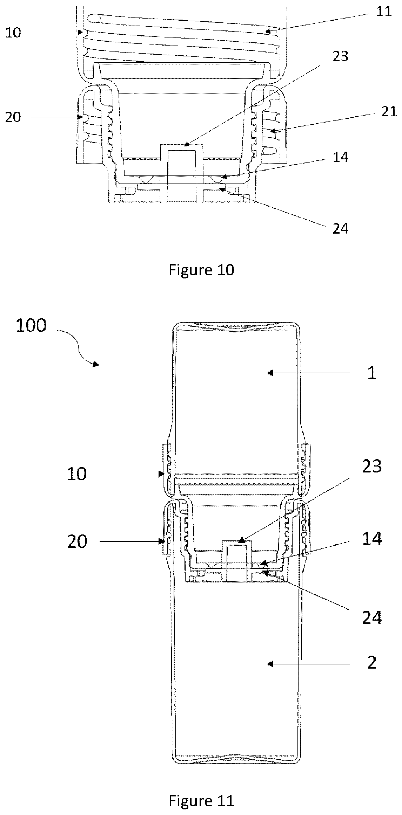

Figure 10 - vertical cross-section of the coupled male cap element (10) and female cap element (20).
Figure 11 - vertical cross-section of the fully closed closure system (100) for processing biological samples.

### DETAILED DESCRIPTION

The more general and advantageous configurations of the present invention are described in the Summary of the Invention. Such configurations are detailed below in accordance with other advantageous and/or preferred embodiments of implementation of the present invention.

In an advantageous aspect of the present invention, the male cap element (10) and the female cap element (20) are produced as single-piece elements by injection molding, in which the membrane (13) is an integral part of the single-piece male cap element (10). Preferably, the thickness of the membrane (13) is configured to limit the rupture potential of the membrane, in which a thicker membrane (13) is able to withstand an increased pressure caused by the perforation structure (23).

In a preferred embodiment of the present invention, the perforation structure (23) at the base of the female cap element (20) comprises a central protruding pillar, and the locking plate (24) is positioned at the bottom and around said central protruding pillar, being the plurality of holes (30) radially positioned at the edge of said locking plate (24). Preferably, the central protruding pillar comprises a cylindrical shape configured to puncture the membrane (13) of the male cap element (10), thus facilitating the rupturing of the membrane (13). In other embodiments of the present invention, the central protruding pillar comprises a pointed tip to facilitate the rupturing of the membrane (13) by increasing the pressure at a given point of contact between the membrane (13) and the central protruding pillar.

In an embodiment of the present invention, the means for coupling (12) with the female cap element (20) comprise external threads, and the means for coupling (22) with the male cap element (10) comprise internal threads. Together, the external threads of the male cap element (10) and the internal threads of the female cap element (20) constitute a rotational closure mechanism that prevents axial movement of the male cap element (10) until a predefined rotational alignment is achieved, and allows the user to manually control the secure engagement of the male cap element (10) with the female cap element (20) by providing progressive axial movement and gradual contact between the central protruding pillar and the rupturable membrane (13) to prevent accidental rupturing of the membrane (13). In addition, the external threads of the male cap element (10) press against the internal threads of the female cap, forming a tight, leak-proof seal to prevent vapor escape and liquid leakage.

In a preferred embodiment of the present invention, the male cap element (10) and the female cap element (20) each comprise gripping means (40), to increase the grip of the user and facilitate the rotation of the male cap element (10) and the female cap element (20) between each other. The gripping means (40) preferably comprise a plurality of grooves or ridges radially positioned along the external surface of the top portion of the open top end of the tubular portion of each male cap element (10) and female cap element (20).

Preferably, the protruding central pillar comprises a maximum height that enables the rupture of the membrane (13) to occur only when the male cap element (10) has been rotated into an intermediate position relative to the female cap element (20) thus preventing accidental rupturing of the membrane (13). In addition, the locking plate (24) preferably locks onto the membrane support structure (14) only when the male cap element (10) has been fully rotated into a final position relative to the female cap element (20). This allows the user to easily identify the rupturing of the membrane (13) and also the successful sealing of the second container (2), when the locking plate (24) locks onto the membrane support structure (14) at a final position of the male cap element (10) and female cap element (20), not allowing further rotation movement and insertion of the male cap element (10) into the female cap element (20).

It is also an object of the present invention a method for assembling and sealing a closure system (100) for processing biological samples, comprising the following steps:
- providing a first container (1) containing a fixative liquid, being said first container (1) sealed with a male cap element (10) having a rupturable membrane (13) at its closed top end and means for coupling (12) with the female cap element (20) on the exterior surface of said male cap element (10);
- providing a second container (2) containing a biological sample, being said second container (2) capped with a female cap element (20) having a perforation structure (23) positioned at the base and means for coupling (22) with the male cap element (10) on the interior surface of said female cap element (20);
- inserting the male cap element (10) into the female cap element (20), engaging the coupling means (12, 22);
- rotating the male cap element (10) relative to the female cap element (20) to progressively insert the male cap element (10) into the female cap element (20) so that the distance between the perforation structure (23) and the membrane (13) is progressively decreased;
- completing the rotation until the male cap element (10) reaches an intermediate position relative to the female cap element (20), causing the perforation structure (23) to puncture the membrane (13), enabling fluid transfer through the plurality of holes (30) from a first container (1) to a second container (2);
- completing the rotation until the male cap element (10) reaches a final position relative to the female cap element (20), causing the locking plate (24) to engage the membrane support structure (14) and sealing the second container (2) and preventing fluid communication between said first (1) and second containers (2).

Of course, the preferred embodiments shown above are combinable, in different possible configurations, being the present invention not limited to the embodiments previously described.

## Claims

1. A closure system (100) for processing biological samples, comprising:
- a first container (1) and a second container (2) configured for fluid storage;
- a closure assembly comprising a single-piece male cap element (10) and a single-piece female cap element (20), said male cap element (10) comprising attaching means (11) for closing said first container (1), means for coupling (12) with the female cap element (20) and one rupturable membrane (13); said female cap element (20) comprising attaching means (21) for closing said second container (2), means for coupling (22) with the male cap element (10), a perforation structure (23) configured to rupture said membrane (13) upon actuation, and a plurality of holes (30) to allow fluid communication between said first (1) and second containers (2); and
- a locking mechanism configured to prevent the fluid communication between said first (1) and second containers (2) upon rupture of said membrane (13), wherein said male cap element (10) comprises a membrane support structure (14) and the female cap element (20) comprises a locking plate (24), being said locking plate (24) configured to lock onto said membrane support structure (14) upon actuation to seal said rupture membrane (13) and block the fluid communication between said first (1) and second containers (2).

2. A closure system according to claim 1, wherein the membrane support structure (14) of the male cap element (10) is permanently connected to the membrane (13) and comprises a rupture zone configured to receive the perforation structure (23) upon the rupturing of the membrane (13).

3. A closure system according to any of the previous claims, wherein:
- the male cap element (10) comprises a first tubular portion with an open top end and a closed bottom end sealed by the rupturable membrane (13), having said means for coupling (12) with the female cap element (20) located on an exterior surface of said first tubular portion;
- the female cap element (20) comprises a second tubular portion with an open top end and a base located at the bottom end, having said means for coupling (22) with the male cap element located on an interior surface of said second tubular portion; and
- the base of the female cap element (20) comprises the perforation structure (23) and said plurality of holes (30) for fluid communication between the first and second containers.

4. A closure system according to any of the previous claims, wherein the perforation structure (23) at the base of the female cap element (20) comprises a central protruding pillar, and the locking plate (24) is positioned at the bottom and around said central protruding pillar, being the plurality of holes (30) radially positioned at the edge of said locking plate (24).

5. A closure system according to claim 4, wherein the central protruding pillar comprises a cylindrical shape.

6. A closure system according to any of the claims 4 - 5, wherein the central protruding pillar comprises a pointed tip configured to puncture the membrane of the male cap element (10).

7. A closure system according to any of the previous claims wherein the means for coupling (12) with the female cap element (20) comprise external threads, and wherein the means for coupling (22) with the male cap element comprise internal threads.

8. A closure system according to any of the claims 4 - 7 wherein the protruding central pillar comprises a maximum height that enables the rupture of the membrane (13) to occur only when the male cap element (10) has been rotated into an intermediate position relative to the female cap element (20).

9. A closure system according to any of the previous claims wherein the locking plate (24) locks onto the membrane support structure (14) only when the male cap element (10) has been fully rotated into a final position relative to the female cap element (20).

10. A closure system according to any of the previous claims wherein the membrane support structure (14) comprises a circular ring.

11. A method for assembling and sealing a closure system for processing biological samples according to claims 1 - 10, comprising the following steps:
- Providing a first container (1) containing a fixative liquid, being said first container (1) sealed with a male cap element (10) having a rupturable membrane (13) at its closed top end and means for coupling (12) with the female cap element (20) on the exterior surface of said male cap element (10);
- Providing a second container (2) containing a biological sample, being said second container (2) capped with a female cap element (20) having a perforation structure (23) positioned at the base and means for coupling (22) with the male cap element (10) on the interior surface of said female cap element (20);
- Inserting the male cap element (10) into the female cap element (20), engaging the coupling means (12, 22);
- Rotating the male cap element (10) relative to the female cap element (20) to progressively insert the male cap element (10) into the female cap element (20) so that the distance between the perforation structure (23) and the membrane (13) is progressively decreased;
- Completing the rotation until the male cap element (10) reaches an intermediate position relative to the female cap element (20), causing the perforation structure (23) to puncture the membrane (13), enabling fluid transfer through the plurality of holes (30) from a first container (1) to a second container (2);
- Completing the rotation until the male cap element (10) reaches a final position relative to the female cap element (20), causing the locking plate (24) to engage the membrane support structure (14) and sealing the second container (2) and preventing fluid communication between said first (1) and second containers (2).
